# EUROPEAN PATENT SPECIFICATION

(11) **EP 1 464 341 B1**
(45) Date of publication and mention of the grant of the patent: **30.05.2012**
(21) Application number: 04101334.3
(22) Date of filing: 31.03.2004
(51) Int. Cl.: A61K 47/22, A61K 9/00

(54) **Ubiquinone-containing water-soluble formulation for ophthalmic use**
Wasserlösliche Zubereitung zur ophthalmische Anwendung, Ubichinon entahltend
Formulation hydrosoluble comprénant de l'ubiquinone pour utilisation oculaire

(30) Priority: 03.04.2003 IT RM20030153
(43) Date of publication of application: 06.10.2004
(73) Proprietor: Visufarma S.p.A, 00191 Roma (IT)
(72) Inventor: CAMPANA, Alberto, 00162, Roma (IT); CAVALLO, Giovanni, 00122, Roma (IT); SODO, Eugenio, 00125, Roma (IT)
(74) Representative: Predazzi, Valentina

(56) References cited:
- EP-A- 1 321 138
- WO-A-01/37851
- WO-A-01/87355
- US-A- 5 558 876
- BRANCATO R ET AL: "PREVENTION OF CORNEAL KERATOCYTE APOPTOSIS AFTER ARGON FLUORIDE EXCIMER LASER IRRADIATION WITH THE FREE RADICAL SCAVENGER UBIQUINONE Q10" EUROPEAN JOURNAL OF OPHTHALMOLOGY, MILAN, IT, vol. 10, no. 1, January 2000 (2000-01), pages 32-38, XP000993572 ISSN: 1120-6721
- ADAMS M W: "D-ALPHA TOCOPHERYL POLYETHYLENE GLYCOL 1000 SUCCINATE (EASTMAN VITAMI E TPGS) AS AN EMULSIFIER AND BIOENHANCER FOR DRUGS AND LIPOPHILIC COMPOUNDS", INTERNATIONAL CONGRESS OF TECHNOLOGY AND PHARMACOLOGY, ASSOC. PHARM. GALENIQUE IND., CHATENAY MALABRY, FR, 1 January 1992 (1992-01-01), pages 254-262, XP000576964,

## Description

The present invention relates to the pharmacological field, and more precisely to the preparations for ophthalmic use.

Object of the invention is a novel formulation containing ubiquinone Q10, or a functionally equivalent derivative thereof, in association with vitamin E TPGS (d-Alpha-tocopheryl polyethylene glycol 1000-succinate), which behaves as an effective solubilizer for the ubiquinone itself. The latter, being hydrophobic, is totally insoluble in water and not useful in the manufacturing of solutions or emulsions for ophthalmic use without resorting to the use of stabilizers and/or surfactants, which however would in turn entail undesired side effects in the ophthalmic use.

Moreover, vitamin E TPGS is apt to act synergistically as antioxidant with ubiquinone.

As functionally equivalent derivatives of the Coenzyme Q10, analogous ubiquinones, as well as the corresponding ubiquinoles being a reduced form thereof, are mentioned hereinafter by way of example.

The formulation according to the present invention can be prepared in the form of aqueous solution, as well as in the form of aqueous emulsion.

Hence, object of the invention is the use of vitamin E TPGS as solubilizer of ubiquinone for the manufacturing of solutions or aqueous emulsions of said ubiquinone.

A further object of the invention is the use of an aqueous solution or emulsion of ubiquinone or an analogous or functionally equivalent derivative thereof in association with vitamin E TPGS for ophthalmic use to reduce the effects of oxidative stresses at the eye level.

Another further object of the invention consists in the use of an aqueous solution or emulsion of ubiquinone or an analogous or functionally equivalent derivative thereof in association with vitamin E TPGS for ophthalmic use for the treatment of the pathological manifestations related to apoptosis on the part of corneal keratocytes.

It has surprisingly being found and experimentally demonstrated, as described in Example 3, that the aqueous solution containing ubiquinone and vitamin E TPGS administered locally on the anterior part of the eye, is rapidly and quantitatively absorbed by the posterior part of the eye itself, so that the ubiquinone may exert its antioxidant and anti-apoptotic action also in correspondence of this district.

Hence, a further aspect of the invention is the use of an aqueous solution or emulsion of ubiquinone or an analogous or functionally equivalent derivative thereof in association with vitamin E TPGS, as an ophthalmic medicament for the treatment of degenerative pathologies comprising ischemic opticopathy, senile opacity of lenses, cataract, uveitis, as well as of heredofamilial, dysmetabolic and senile age-related degenerative pathologies, and of neurodegenerative pathologies of the retina and of the optical nerve comprising glaucoma, senile macular degeneration, retinitis pigmentosa, Stargardt disease, vitelliform macular cysts, cones dystrophy, proliferative diabetic retinopathy, hypertensive retinopathy, retinal detachment, retinoblastoma, as well as neuritis and optical neuropathies of toxic, inflammatory and degenerative origin.

A further aspect and an object of the invention is an aqueous solution or emulsion for ophthalmic use containing ubiquinone from 0.05 to 0.5 percent of the total weight and vitamin E TPGS from 0.05 to 10 percent of the total weight, as well as pharmaceutically compatible excipients.

According to the invention, the antioxidant action exerted by ubiquinone and by Vitamin E TPGS can be further enhanced by the addition of a further water-soluble antioxidant, represented by an ascorbyl derivative: the Magnesium ascorbyl-2-phosphate.

Additionally or alternatively to the latter there may be used other antioxidants, naturally present in the lachrymal fluid, like cysteine and tyrosine.

Further components may be additioned to the preparation object of the invention, in order to attain both a suitable viscosity and a suitable isotony thereof.

The aqueous solution or emulsion for ophthalmic use according to the invention is apt to preserve and improve the physiology of the tissues of the eye, and in particular of the corneal tissue, as well as to restore the normal conditions thereof following traumas, surgery or pathological events, as it is capable of inhibiting the pathological manifestations due to an excessive apoptosis on the part of corneal keratocytes and of reducing the effects of the free radicals, detrimental to the normal eye physiology.

Therefore, the aqueous solution or emulsion according to the invention can be useful as pharmaceutically active agent for the treatment of the above-mentioned disorders.

Further features and advantages of the invention will be made apparent in the following description.

### Overview of the Prior Art

Ubiquinone Q10 (also denominated Ubidecarenone or Coenzyme Q10) chemically consists of 2,3-dimethoxy-5-methylbenzoquinone to which a terpenoid chain, consisting of ten monounsaturated trans-isoprenoid units, is attached. Said molecule is lipophilic and totally insoluble in water.

Ubiquinol, which is a reduced form of ubiquinone, exhibits the same antioxidant properties of ubiquinone and can therefore replace ubiquinone for the purposes of the present invention.

Human cells synthesize ubiquinone Q10 from the amino acid tyrosine, via an 8-step process similar to cholesterol synthesis; the synthesis of the terpenoid chain requires the presence of suitable levels of folic acid, niacin and vitamins B2, B6 and C.

In humans the hematic concentration of said molecule, under normal conditions, is of 0.7-1.0 µg/ml, although it decreases with age, owing to its reduced synthesis and the concomitant increase of the lipid peroxidation.

At the eye level, it is known the action of ubiquinone as scavenger with regard to the free radicals (Brancato, R., Schiavone, N., Siano, S., Lapucci, L., Papucci, L., Donnini, M., Formigli, L., Orlandini, S.Z., Carella, G., Carones, F. and Capaccioli, S., 2000, Eur. Ophthalmol., 10:32-28 describing the prevention of corneal keratocyte apoptosis after argon fluoreide excimer laser irradiation with the free radical scavenger ubiquinone Q10; Blasi, M.A., Bovina, C., Carella, G., Geneva, M.L., Jansen, A.M.,Lenaz, G. and Brancato, R., 2001, Ophthalmologica, 215:51-54; Brancato, R., Fiore, T., Papucci, L., Schiavone, N., Formigli, L., Orlandini, S.Z., Gobbi, P.G., Carones, F., Donnini, M., Lapucci, A. and Capaccioli, S., 2002, J. Refract. Surg., 18:135-139). Moreover, there has been highlighted the key role of ubiquinone in the mitochondrial respiratory chain, both as a structural and as a modulator element of the permeability of the mitochondrial pores, whose opening alters the transmembrane electrochemical gradient and causes the extrusion of the cytochrome-c into the cytoplasm. In particular, the cytochrome-c binds the Apaf-1 (apoptotic protease activating factor-1) and activates the caspase-dependant apoptotic process leading to the release of free radicals, like the reactive oxygen species (ROS).

Recently, it has been highlighted that tocopherol (vitamin E) is capable of acting synergistically with ubiquinone, enhancing the antiapoptotic effects of the latter by inhibiting the production of free radicals and/or adjusting the permeability of the mitochondrial pore. In fact, the excessive apoptosis on the part of corneal keratocytes that, by activating tissue repair, causes an abnormal deposition of collagen, proteoglycans and ialuronic acid, is an undesired effect of the photoreactive keratectomy (PRK) surgery with excimer laser (one of the surgical methods most widely used to correct myopia, astigmatism and hypermetropia) and may cause a visual haze, regression of the correction to the corneal curvature, light-scattering and the entailed worsening of the sight.

The apoptosis of the stromal keratocytes can also be caused by chemical agents (e.g., genotoxic drugs), physical agents (like UV light, radiations or mechanical insults), biologic agents (like the herpes virus) and also ocular pathologies like the age-related macular degeneration (AMD) (Brancato, R., Fiore, T., Papucci, L., Schiavone, N., Formigli, L., Orlandini, S.Z., Gobbi, P.G., Carones, F., Donnini, M., Lapucci, A., Capaccioli, S., 2002, J. Refract. Surg., 18:135-139). In ophthalmology it is known the use of ubiquinone, also in combination with tocopherol (vitamin E), for the topical treatment of the eye following surgery as well as pathologies like the above-mentioned ones (EP 1 231 909). Moreover, it has been demonstrated that ubiquinone and tocopherol act synergistically as antioxidants, in particular on lipid peroxidase (Niki, E., 1997, Molec. Aspects Med, 18 (Suppl): s63-s70).

The patent application WO01/37851 discloses the use of coenzyme Q10 solubilised by tocopherol for the local treatment of ophthalmologic pathologies following photoreactive therapy, refractive surgery and exposure to ultraviolet radiation.

The patent application WO 01/87355 discloses oral formulations of coenzyme Q10 and in example 1 describes an emulsion comprising of 10% Vitamin E TPGS

### DETAILED DESCRIPTION OF THE INVENTION

The main difficulty found in the manufacturing of ubiquinone-containing aqueous solutions is that of solubilizing said hydrophobic molecule. In the above-cited EP 1 231 909, ubiquinone is kept in aqueous solution by virtue of the presence of a stabilizing copolymer (10-15% Lutrol 127), and of a powerful nonionic surfactant (5% Cremophor), which are both in quite high percents. The sterility of the solution is ensured by the presence of a preservative such as benzalkonium chloride (0.010).

### DETAILED DESCRIPTION OF THE INVENTION

The main difficulty found in the manufacturing of ubiquinone-containing aqueous solutions is that of solubilizing said hydrophobic molecule. In the above-cited EP 1 231 909, ubiquinone is kept in aqueous solution by virtue of the presence of a stabilizing copolymer (10-15% Lutrol 127), and of a powerful nonionic surfactant (5% Cremophor), which are both in quite high percents. The sterility of the solution is ensured by the presence of a preservative such as benzalkonium chloride (0.010).

However, said preparation, as well as many others available on the market, entails several problems and limitations. First of all, there has to be stressed the difficulty of its production on an industrial scale, due to the high viscosity of its components. Moreover, it is well-documented that the use of solubilizers like Lutrol and Cremophor can foster the onset of undesired eye hydration phenomena and the entailed alteration of the corneal epithelium (Monti, D., Chetoni, P., Burgalassi, S., Najarro, M. and Saettone, M.F., 2002, Int. J. Pharm., 232:139-147). Lastly, it has been demonstrated that Lutrol 127 can cause serious damages to the retina (Davidorf, F.H, Chambers, R.B., Kwon, O.W., Doyle, W., Gresak, P. and Frank, S.G., 1990, Retina, 10:297-300).

Hence, there remained unsolved the problem of bringing the ubiquinone in an aqueous solution satisfactory from the viewpoint of a topical application directly into contact with the eye, in particular in the form of collyrium.

According to the present invention the problems related to the manufacturing of ubiquinone-based ophthalmic solutions have surprisingly been solved, using in association with the ubiquinone a form of vitamin E, different from tocopherol, capable of making the Coenzyme Q10 soluble in aqueous solution without use of further solubilizers and/or stabilizers.

More precisely, according to the present invention, it has been implemented the solubilization of ubiquinone directly with the substance identified in short as `vitamin E TPGS', i.e. according to chemical nomenclature, d-Alpha-tocopheryl polyethylene glycol 1000-succinate.

Vitamin E TPGS may be considered as a water-soluble form of vitamin E of natural origin, which is obtained by esterifying the acid group of the d-Alpha-tocopheryl succinic acid with polyethylene glycol (PEG) 1000.

Such a molecule entails several features making it advantageously useful for the manufacturing of ophthalmic solutions, and precisely:
- vitamin E TPGS exhibits an HBL *(Hydrophile*/ *Lipophile Balance*) value equal to 13.5 and is capable of effectively solubilizing lipophilic substances, ubiquinone included. The former, exhibiting no unstability and toxicity problems, behaves as a very valid and safe ophthalmic medium for the latter;
- vitamin E TPGS is very stable, as it undergoes no hydrolysis when stored, even for a very long time;
- vitamin E TPGS is a source of bioavailable tocopherol;
   (1 g vitamin E TPGS converts, by virtue of the action of esterase, into about 260 mgr tocopherol);
- vitamin E TPGS is an amphipatic molecule, i.e. lipophilic as well as hydrophilic, and it behaves as a typical smrfactant.

The combination of these features proves effective, according to the invention, to solve the hereto-described problems, associated to the use of ubiquinone in aqueous solution for a therapeutic treatment into direct contact with the eye.

Hence, the preparation object of the present invention, containing ubiquinone and vitamin E TPGS, may effectively be employed to inhibit the keratocyte apoptosis and the negative effects of the free radicals at the eye level, without however exhibiting the typical side effects due to the use of solubilizers and stabilizers, required to keep the ubiquinone in solution.

An additional feature of the invention is that of providing also the addition, to the ubiquinone/vitamin E TPGS formulation, of a further water-soluble antioxidant, in the form of ascorbyl-2-phosphate and/or of the magnesium salt thereof. In fact, it is well-known that to insults leading to the production of free radicals in the human organism there is associated a concomitant and marked reduction in the levels of ubiquinone, tocopherol and ascorbic acid (Yamamoto, Y., 2001, J. Dermatol Sci., 27 (suppl 1: Sl-S4; Lemke, M., Frei, B., Ames, B.N. and Faden, A.I., 1990, Neurosci Lett., 108:201-206).

The ascorbyl-2-phosphate molecule is capable of enhancing the antioxidant effect of the ubiquinone/vitamin E TPGS system (Schneider, D. and Elstner, E.F., 2000, Antioxid Redox Signal, 2:327-333; Weber, C., Sejersgard Jacobsen, T., Mortensen, S.A., Paulsen, G. and Holmer, G., 1994, Int. J. Vitamin. Nutr. Res., 64:311-315; Mukai, K., Itoh, S. and Morimoto, H., 1992, J. Biol. Chem., 267:2227722281).

Moreover, recently there has been demonstrated a marked reduction of the ascorbic acid in the lachrymal fluid following surgery (PRK), traumas or proinflammatory insults, suggesting the use of said molecule in the therapeutic field (Bilgihan, A., Bilgihan, K., Toklu, Y., Konuk, O., Yis, O. and Hasanreisoglu, B., 2001, J. Cataract. Refract. Surg., 27:585-588). With respect to the ascorbic acid, which is the main antioxidant present in the aqueous phases of the eye (lachrymal fluid, aqueous humour and vitreous body), there has been highlighted the advantage of using Magnesium ascorbyl-phosphate, which is much more stable in aqueous solution. In fact, Mg ascorbyl phosphate does not oxidize to dehydroascorbic acid and moreover it does not exhibit a marked effect on the pH of the solution (Choy, C.K., Benzie, I.F. and Cho, P., 2000, Invest. Ophthalmol. Vis. Sci., 41:3293-3298; Hanashima, C. and Namiki, H., 1999, Cell. Biol. Int., 23:287-298).

Besides from ascorbyl-derivatives, there is proposed the addition into the ophthalmic solution of other water-soluble antioxidants, usually present in the lachrymal fluid, like cysteine and tyrosine (Gogia, R., Richer, S.P. and Rose, R.C., 1998, Curr. Eye Res., 17:257-263). According to the invention the ophthalmic solution may further be additioned with some excipients:
- hydroxymethyl cellulose or hydroxypropyl cellulose to provide a suitable viscosity;
- mannitol or NaCl to attain the right isotony;
- almond oil, rice oil, soy oil to prepare the same composition in the form of emulsion;
- benzalkonium chloride, as preservative, to ensure sterility.

In the testing carried out there has been singled out a preferred formulation of the aqueous solution containing ubiquinone in a percent by weight equal to the 0.05% - 0.5% and vitamin E TPGS in a percent by weight equal to the 0.05% - 10%.

In a further embodiment ascorbyl-phosphate was added, also in the form of Magnesium salt, in a percent by weight equal to the 0.05% - 1%. To these formulations there were added water-soluble antioxidants (cysteine and tyrosine) in a percent by weight not higher than the 1%.

As excipients there were additioned the following components:
- hydroxymethyl cellulose or hydroxypropyl cellulose in a percent by weight not higher than the 1% ;
- mannitol or NaC1 in a percent by weight not higher than the 1%.
- boric acid or sodium borate in a percent by weight not higher than the 1%.
- disodium edetate (EDTA) in a percent by weight not higher than the 0.1%.
- benzalkonium chloride in a percent by weight not higher than the 0.01%.

In order to make available said preparation in the form of emulsion, emulsioning oils (almond oil, rice oil, soy oil) were added in a percent by weight not higher than the 5%.

Hereinafter there are shown some exemplary embodiments of aqueous solutions manufactured according to the above-described formulation.

| **FORMULATION 1** **Ingredient** | **Concentration** **(% w/v)** |
|---|---|
| Ubiquinone | 0.20 |
| Vitamin E TPGS | 5.0 |
| NaCl | q.s. to isotony |
| Benzalkonium chloride | 0.01 |
| Bidistilled water | q.s. to 100.00 ml |

| **FORMULATION 2** **Ingredient** | **Concentration** **(% w/v)** |
|---|---|
| Ubiquinone | 0.20 |
| Vitamin E TPGS | 5.0 |
| Mannitol | q.s to isotony |
| Benzalkonium chloride | 0.01 |
| Bidistilled water | q.s. to 100.00 ml |

| **FORMULATION 3** **Ingredient** | **Concentration** **(% w/v)** |
|---|---|
| Ubiquinone | 0.40 |
| Vitamin E TPGS | 10.0 |
| NaCl | q.s to isotony |
| Benzalkonium chloride | 0.01 |
| Bidistilled water | q.s. to 100.00 ml |

| **FORMULATION 4** **Ingredient** | **Concentration** **(% w/v)** |
|---|---|
| Ubiquinone | 0.40 |
| Vitamin E TPGS | 10.0 |
| Mannitol | q.s. to isotony |
| Benzalkonium chloride | 0.01 |
| Bidistilled water | q.s. to 100.00 ml |

| **FORMULATION 5** **Ingredient** | **Concentration** **(% w/v)** |
|---|---|
| Ubiquinone | 0.20 |
| Vitamin E TPGS | 5.0 |
| Mg ascorbyl-2-phosphate | |
| NaCl | q.s. to isotony |
| Benzalkonium chloride | 0.01 |
| Bidistilled water | q.s. to 100.00 ml |

| **FORMULATION 6** **Ingredient** | **Concentration** **(% w/v)** |
|---|---|
| Ubiquinone | 0.20 |
| Vitamin E TPGS | 5.0 |
| Mg ascorbyl-2-phosphate | 0.2 |
| Mannitol | q.s. to isotony |
| Benzalkonium chloride | 0.01 |
| Bidistilled water | q.s. to 100.00 ml |

| **FORMULATION 7** **Ingredient** | **Concentration** **(% w/v)** |
|---|---|
| Ubiquinone | 0.40 |
| Vitamin E TPGS | 10.0 |
| Mg ascorbyl-2-phosphate | 0.2 |
| NaCl | q.s to isotony |
| Benzalkonium chloride | 0.01 |
| Bidistilled water | q.s. to 100.00 ml |

| **FORMULATION 8** **Ingredient** | **Concentration** **(% w/v)** |
|---|---|
| Ubiquinone | 0.40 |
| Vitamin E TPGS | 10.0 |
| Mg ascorbyl-2-phosphate | 0.2 |
| Mannitol | q.s. to isotony |
| Benzalkonium chloride | 0.01 |
| Bidistilled water | q.s. to 100.00 ml |

| **FORMULATION 9** **Ingredient** | **Concentration** **(% w/v)** |
|---|---|
| Ubiquinone | 0.20 |
| Vitamin E TPGS | 5.0 |
| Sodium chloride | q.s to isotony |
| Benzalkonium chloride | 0.01 |
| Bidistilled water | q.s. to 100.00 ml |
| Boric acid | 0.775 |
| Sodium borate | 0.125 |
| Disodium EDTA | 0.100 |
| pH | 7.22 |

| **FORMULATION 10** **Ingredient** | **Concentration** **(% w/v)** |
|---|---|
| Ubiquinone | 0.40 |
| Vitamin E TPGS | 5.0 |
| Sodium chloride | q.s to isotony |
| Benzalkonium chloride | 0.01 |
| Bidistilled water | q.s. to 100.00 ml |
| Boric acid | 0.775 |
| Sodium borate | 0.125 |
| Disodium EDTA | 0.100 |
| pH | 7.22 |

| **FORMULATION 11** **Ingredient** | **Concentration** **(% w/v)** |
|---|---|
| Monosodium phosphate dihydrate | 0,220 |
| Disodium phosphate dihydrate | 0,950 |
| Sodium chloride | q.s. to isotony |
| Vitamin E TPGS | 8,0 |
| Ubiquinone | 0,2 |
| Deionized water | q.s. to 100,00 ml |

Hence, from the above it is evident that the main innovative feature of the present invention is the use of vitamin E TPGS associating to its normal therapeutical effects the capacity of acting as an effective solubilizer for ubiquinone (as it has an HBL *(Hydrophile*/*Lipophile Balance)* favourable to the solubilization of the lipophilic substances), making said preparation not only easy to manufacture industrially, but also free from undesired side effects related to the use of stabilizers and/or surfactants like Lutrol 127 and/or Cremophor.

A further innovative feature of the invention is the / addition of a further water-soluble antioxidant, represented by an ascorbyl-derivative, like the Magnesium ascorbyl-2-phosphate, to exploit the synergy among ubiquinone, tocopherol and ascorbic acid with reference to their antioxidant power; it also has to be highlighted that the presence of vitamin E TPGS, determines a low viscosity of the formulation, which therefore is more comfortable for the use with respect to very viscous formulations. The low viscosity of the solution, due to the presence of vitamin E TPGS, significantly facilitates its production on an industrial scale, as it allows also a single-dose preparation, with no addition of preservatives; and lastly, *in vivo* there has been demonstrated the scavenging action of vitamin E TPGS with regards to the superoxide anion generated by human PMNs (polymorphonucleates) suitably stimulated (Ferrocytochrome C method).

Hereinafter, there are described testings on the tolerability of the topical administration of a Coenzyme Q10- and Vitamin E TPGS-based ophthalmic solution according to the invention.

### Example 1

*Acute local tolerability testing of a Coenzyme Q10-and Vitamin E TPGS-based ophthalmic product following single administration in rabbit.*

There was conducted a local tolerability study for a 0.1%, 0.2% and 0.4% Coenzyme Q10-based ophthalmic product (collyrium) following single administration in rabbit.

The products exhibited the following percent by weight composition:

| Product | **A** | **B** | **C** |
|---|---|---|---|
| Boric acid | 0.775 | 0.775 | 0.775 |
| Sodium borate | 0.125 | 0.125 | 0.125 |
| Disodium EDTA | 0.100 | 0.100 | 0.100 |
| Sodium chloride | 0.250 | 0.250 | 0.250 |
| N-hydroxymethylglycinate | 0.020 | 0.020 | 0.020 |
| Vitamin E TPGS | 6.000 | 6.000 | 6.000 |
| Coenzyme Q10 | 0.100 | 0.200 | 0.400 |
| Deionized water | q.s. to 100 ml | | |
| pH | 7.25 | 7.19 | 7.22 |
| mOsm/kg | 295 | 306 | 301 |

The placebo was a product of analogous composition, with the difference that it contained neither Co Q10, nor vitamin E TPGS.

There was conducted a local tolerability study for a 0.1%, 0.2% and 0.4% Coenzyme Q10-based ophthalmic product (collyrium) following single administration in rabbit.

The New Zealand rabbits in the study were divided into three experimental groups, each consisting of 3 animals, to which a constant volume of 0.1 ml of respectively 0.1%, 0.2% and 0.4% Coenzyme Q10-based ophthalmic preparation (collyrium) was administered in the conjunctival sac of the right eye. A constant volume of 0.1 ml of the related placebo was administered in the conjunctival sac of the left eye.

Observations were conducted to detect any ocular irritation at +0.5, 2, 4, 24, 48 and 72 h from the single administration. No signs of local irritation were detected in the eyes treated with the substances under exam.

### EXAMPLE 2

*Acute local tolerability testing of a Coenzyme Q10-and Vitamin E TPGS-based ophthalmic product following administration repeated for 28 days in rabbit.*

With the same products used in the Example 1 there was conducted a local tolerability study for a Coenzyme 0.1%, 0.2% and 0.4% Q10-based ophthalmic preparation (collyrium) in rabbit following ocular administration repeated for 28 days, twice per day. The New Zealand rabbits in the study were divided into three experimental groups, each consisting of 3 animals, to which a constant volume of 0.1 ml of respectively 0.1%, 0.2% and 0.4% Coenzyme Q10-based ophthalmic preparation (collyrium) was administered in the conjunctival sac of the right eye. In the conjunctival sac of the right eye of each rabbit 0.1 ml of the related product A, B or C, and in the left eye 0.1 ml of the placebo, respectively, were administered twice per day, for 28 days. Daily, observations were conducted to detect any ocular irritation. At the end of the treatment the animals were sacrificed and the harvested eyes were subjected to the histological examination of the following tissues: cornea, conjunctive, retina, nictitating membrane, lachrymal gland.

Under the above-indicated experimental conditions, the substances being examined demonstrated to cause no ocular irritation in rabbit, following ocular administration repeated twice per day for 28 days.

### Example 3

*Assessment of the pharmacokinetic activity of incorporation of the hydrophilic collyrium in rabbit cornea, retina and plasma.*

a) The hydrophilic collyrium was assayed on a first lot of three rabbits by instilling Coenzyme Q10 in the right eye and an equivalent excipient free from Q10 and vitamin E TPQS in the contralateral (basal) eye. At + 15 min from the collyrium instillation, CoQ10 concentration in the treated eye and in the other (basal) eye was analysed. The analysis was performed on the retina, the cornea and the plasma of the two eyes.

The results of the assay are reported in the following

**Table 1**

| 3 rabbits N. | RETINA (pmoles/g) 15 min | | | |
|---|---|---|---|---|
| | Basal | Q10- treated | Difference | %Increase |
| 15 | 868 | 787 | -82 | -9 |
| 16 | 618 | 632 | 14 | 2 |
| 17 | 760 | 843 | 83 | 11 |
| Average | 769 | 754 | 5 | 1 |
| Standard deviation | 126 | 109 | | |
| | | | | |

| 3 rabbits N. | CORNEA(pmoles/g) 15 min | | | |
|---|---|---|---|---|
| | Basal | Q10- treated | Difference | %Increase |
| 15 | 921 | 887 | -34 | -3 |
| 16 | 578 | 955 | 377 | 65 |
| 17 | 1320 | 1170 | -150 | -11 |
| Average | 940 | 1004 | 74 | 7 |
| Standard deviation | 371 | 148 | | |
| | | | | |

| 3 rabbits N. | PLASMA (pmoles/g) 15 min | | | |
|---|---|---|---|---|
| | Basal | Q10-treated | Difference | %Increase |
| 15 | 54 | 62 | 8 | 15 |
| 16 | 77 | 83 | 6 | 8 |
| 17 | 61 | 78 | 17 | 28 |
| Average | 64 | 74 | 10 | 16 |
| Standard deviation | | | | |

The assay shows a ready incorporation into the plasma already at +15 min from the administration, with a 16% average increase with respect to the basal.

b) .A second lot of six rabbits was subjected to an analogous assay in which the waiting time was prolonged up to 30 min.

The results are reported in the following

**TABLE 2**

| 6 rabbits N. | RETINA (pmoles/g) 15 min | | | |
|---|---|---|---|---|
| | Basal | Q10-treated | Difference | % Increase |
| 18 | 829 | 1006 | 177 | 21 |
| 19 | 1337 | 1394 | 57 | 4 |
| 20 | 931 | 1037 | 106 | 11 |
| 21 | 651 | 725 | 74 | 11 |
| 22 | 754 | 975 | 211 | 28 |
| 23 | 1161 | 1369 | 208 | 18 |
| Average | 946 | 1084.3 | 138.8 | 15 |
| Standard Deviation | 258 | 255 | | |
| | | | | |

| 6 rabbits N. | CORNEA (moles/g) 30 min | | | |
|---|---|---|---|---|
| | Basal | Q10-treated | Difference | %Increase |
| 18 | 675 | 560 | -115 | -17 |
| 19 | 701 | 817 | 116 | 16 |
| 20 | 1237 | 1434 | 197 | 16 |
| 21 | 801 | 991 | 190 | 24 |
| 22 | 977 | 970 | -7 | -7 |
| 23 | 931 | 778 | -153 | -16 |
| Average | 887 | 925 | 38 | 4 |
| Standard Deviation | 210 | 294 | | |
| | | | | |

| 6 rabbits N. | PLASMA (pmoles/g) 30 min | | | |
|---|---|---|---|---|
| | Basal | Q10-treated | Difference | %Increase |
| 18 | 65 | 79 | 14 | 21 |
| 19 | 98 | 105 | 7 | 7 |
| 20 | 83 | 106 | 23 | 28 |
| 21 | 43 | 53 | 10 | 23 |
| 22 | 67 | 81 | 14 | 21 |
| 23 | 65 | 73 | 8 | 12 |
| Average | 70 | 83 | 13 | 18 |
| Standard Deviation | | | | |

The results show that in the retina there is a significant incorporation at +30 min only (average 15% increase with respect to the contralateral eye).

The incorporation in the cornea is scarce at both times considered.

Since for the incorporation in the retina the right eye is compared to the eye of the same animal, there can be concluded that the collyrium is uptaken directly in a transocular manner and not via the hematic route.

Surprisingly, the experimental result confirms the fact that the topical administration directly into the eye of the aqueous solution or emulsion of CoQ10 according to the invention, is capable of bringing the active therapeutic agent directly into contact with the district of interest with no need to pass through the hematic route, which would reduce its effectiveness.

This means that the therapeutic agent Co Q10 carried by the aqueous solution or emulsion containing Vitamin E TPGS is active both towards the anterior part of the eye (cornea, iris, conjunctival sac) and the posterior part of the eye (retina, optic nerve), in contrast with the contrary opinion of the common teaching of the prior art.

Hence, it can be used for the treatment of degenerative and neurodegenerative pathologies of the eye, particularly those involving PCD phenomena (apoptosis) .

Among these pathologies, by way of example, there are indicated the following:
oxidative stresses at the eye level, pathological manifestations related to the altered apoptosis on the part of corneal keratocytes, degenerative pathologies comprising ischemic opticopathy, senile opacity of lenses, cataract, uveitis, as well as heredofamilial, dysmetabolic and age-related degenerative pathologies, and neurodegenerative pathologies of the retina and of the optic nerve comprising glaucoma, senile macular degeneration, retinitis pigmentosa, Stargardt disease, vitelliform macular cysts, cones dystrophy, proliferative diabetic retinopathy, hypertensive retinopathy, retinal detachment, retinoblastoma, as well as neuritis and optical neuropathies of toxic, inflammatory and degenerative origin.

## Claims

1. Use of vitamin E TPGS as solubilizer of ubiquinone or ubiquinol, for the manufacturing of aqueous solutions or aqueous emulsions of said ubiquinone or ubiquinol.

2. An aqueous solution or emulsion of ubiquinone or ubiquinol, in association with vitamin E TPGS, for use as an ophthalmic drug to reduce the effects of oxidative stresses at the eye level.

3. An aqueous solution or emulsion of ubiquinone or ubiquinol, in association with vitamin E TPGS, for use as an ophthalmic drug for the treatment of the pathological manifestations related to the apoptosis on the part of corneal keratocytes.

4. An aqueous solution or emulsion of ubiquinone or ubiquinol, in association with vitamin E TPGS, for use as an ophthalmic drug for the treatment of degenerative pathologies comprising ischemic opticopathy, senile opacity of lenses, cataract, uveitis, as well as of heredofamilial, dysmetabolic, and senile age-related degenerative pathologies.

5. An aqueous solution or emulsion of ubiquinone or ubiquinol, in association with vitamin E TPGS, for use as an ophthalmic drug for the treatment of neurodegenerative pathologies of the retina and of the optic nerve comprising glaucoma, senile macular degeneration, retinitis pigmentosa, Stargardt disease, vitelliform macular cysts, cones dystrophy, proliferative diabetic retinopathy, hypertensive retinopathy, retinal detachment, retinoblastoma, as well as neuritis and optical neuropathies of toxic, inflammatory and degenerative origin.

6. An aqueous solution or emulsion of ubiquinone or ubiquinol, in association with vitamin E TPGS, for use as an ophthalmic drug according to any one of claims 2 to 5, wherein said ubiquinone is Coenzyme Q10 or the corresponding ubiquinol.

7. An aqueous solution for ophthalmic use according to any one of the claims 2 to 6, containing ubiquinone from 0.05 to 0.5 percent of the total weight and vitamin E TPGS from 0.05% to 10% percent of the total weight, as well as pharmaceutically compatible excipients.

8. An aqueous solution for ophthalmic use according to claim 7, having the following percent composition on the total weight:
| | |
|---|---|
| Ubiquinone | 0.05 - 0.5 |
| vitamin E TPGS | 0.05 - 10 |
| Mg-ascorbyl-phosphate | 0.05 - 1 |

9. An aqueous solution for ophthalmic use according to claim 7 or 8, having the following percent composition on the total weight:
| | |
|---|---|
| ubiquinone | 0.05 - 0.5 |
| vitamin E TPGS | 0.05 - 10 |
| Mg-ascorbyl-phosphate | 0.05 - 1 |
| water-soluble antioxidants | ≤ 1 |

10. An aqueous solution for ophthalmic use according to claim 9, wherein the water-soluble antioxidants are cysteine and/or tyrosine.

11. An aqueous solution for ophthalmic use according to any one of claims 7 to 10, having the following percent composition on the total weight:
| | |
|---|---|
| ubiquinone | 0.05 - 0.5 |
| vitamin E TPGS | 0.05 - 10 |
| Mg-ascorbyl-phosphate | 0.05 - 1 |
| water-soluble antioxidants | ≤ 1 |
| hydroxymethyl cellulose | ≤ 1 |

12. An aqueous solution for ophthalmic use according to claim 11, wherein hydroxymethyl cellulose is replaced by hydroxypropyl cellulose.

13. An aqueous solution for ophthalmic use according to any one of claims 7 to 12, having the following percent composition on the total weight:
| | |
|---|---|
| ubiquinone | 0.05 - 0.5 |
| vitamin E TPGS | 0.05 - 10 |
| Mg-ascorbyl-phosphate | 0.05 - 1 |
| water-soluble antioxidants | ≤ 1 |
| hydroxymethyl cellulose | ≤ 1 |
| mannitol | ≤ 1 |

14. An aqueous solution for ophthalmic use according to claim 13, wherein mannitol is replaced by sodium chloride (NaCl).

15. An aqueous solution for ophthalmic use according to any one of claims 7 to 13 having the following percent composition on the total weight:
| | |
|---|---|
| ubiquinone | 0.05 - 0.5 |
| vitamin E TPGS | 0.05 - 10 |
| Mg-ascorbyl-phosphate | 0.05 - 1 |
| water-soluble antioxidants | ≤ 1 |
| hydroxymethyl cellulose | ≤ 1 |
| mannitol | ≤ 1 |
| benzalkonium chloride | ≤ 0.01 |

16. An aqueous solution for ophthalmic use according to any one of claims 7 to 14, having the following percent composition on the total weight:
| | |
|---|---|
| Ubiquinone | 0.05 - 0.5 |
| vitamin E TPGS0, | 05 - 10 |
| Mg-ascorbyl-phosphate | 0.05 - 1 |
| water-soluble antioxidants | ≤ 1 |
| sodium chloride | ≤ 1 |
| benzalkonium chloride | ≤ 0.01 |
| boric acid and/or sodium borate ≤ | 0.1 |
| disodium edetate | ≤ 0.1 |

17. Emulsion for ophthalmic use according to any one of claims 1 to 4, having the following percent composition on the total weight:
| | |
|---|---|
| ubiquinone | 0,05 - 0.5 |
| vitamin E TPGS | 0,05 - 10 |
| Mg-ascorbyl-phosphate | 0.05 - 1 |
| water-soluble antioxidants | ≤ 1 |
| hydroxymethyl cellulose | ≤ 1 |
| mannitol | ≤ 1 |
| vegetable oils | ≤ 5 |

18. Emulsion for ophthalmic use according to claim 16 wherein the vegetable oils are almond oil, rice oil, soy oil.

19. Use of ubiquinone or ubiquinol in association with vitamin E TPGS according to any one of the claims 2 to 4, for the manufacturing of an aqueous solution for ophthalmic use as described in any one of the claims 7 to 16.

20. Use of ubiquinone or ubiquinol in association with vitamin E TPGS according to any one of the claims 2 to 4, for the manufacturing of an aqueous emulsion for ophthalmic use as described in any one of the claims 17 or 18.

## Patentansprüche

1. Verwendung von Vitamin E TPGS als Lösungsvermittler von Ubichinon oder Ubichinol zur Herstellung von wässrigen Lösungen oder wässrigen Emulsionen des Ubichinons oder Ubichinols.

2. Eine wässrige Lösung oder Emulsion von Ubichinon oder Ubichinol in Verbindung mit Vitamin E TPGS zur Verwendung als ophthalmisches Arzneimittel, um die Auswirkungen von oxidativem Stress im Augenbereich zu reduzieren.

3. Eine wässrige Lösung oder Emulsion von Ubichinon oder Ubichinol in Verbindung mit Vitamin E TPGS zur Verwendung als ophthalmisches Arzneimittel zur Behandlung der pathologischen Symptome, die im Zusammenhang mit Apoptose auf dem Teil von Hornhautkeratozyten stehen.

4. Eine wässrige Lösung oder Emulsion von Ubichinon oder Ubichinol in Verbindung mit Vitamin E TPGS zur Verwendung als ophthalmisches Arzneimittel zur Behandlung von degenerativen Pathologien, umfassend ischämische Optikopathie, senile Trübung der Linsen, grauer Star, Uveitis, sowie von heredofamiliären, dysmetabolischen und senilen altersbedingten degenerativen Pathologien.

5. Eine wässrige Lösung oder Emulsion von Ubichinon oder Ubichinol in Verbindung mit Vitamin E TPGS zur Verwendung als ophthalmisches Arzneimittel zur Behandlung von neurodegenerativen Pathologien der Retina und des Sehnervs, umfassend Glaukom, senile Makulardegeneration, Retinitis Pigmentosa, Morbus Stargardt, vitteliforme Makulazysten, Zapfendystrophie, proliferative diabetische Retinopathie, hypertensive Retinopathie, Netzhautablösung, Retinoblastom, sowie Nervenentzündung und optische Neuropathien, welche toxischen, entzündlichen und degenerativen Ursprung haben.

6. Eine wässrige Lösung oder Emulsion von Ubichinon oder Ubichinol in Verbindung mit Vitamin E TPGS zur Verwendung als ophthalmisches Arzneimittel gemäß einem der Ansprüche 2 bis 5, wobei das Ubichinon Coenzym Q10 oder das korrespondierende Ubichinol ist.

7. Eine wässrige Lösung zur ophthalmischen Verwendung gemäß einem der Ansprüche 2 bis 6, die 0,05 bis 0,5 Prozent des Gesamtgewichts an Ubichinon und 0,05% bis 10% Prozent des Gesamtgewichts an Vitamin E TPGS sowie pharmazeutisch verträgliche Hilfsstoffe enthält.

8. Eine wässrige Lösung zur ophthalmischen Verwendung gemäß Anspruch 7, welche die folgende Zusammensetzung in Prozent auf das Gesamtgewicht aufweist:
| | |
|---|---|
| Ubichinon | 0,05 - 0,5 |
| Vitamin E TPGS | 0,05 - 10 |
| Mg-Ascorbyl-phosphat | 0,05 - 1. |

9. Eine wässrige Lösung zur ophthalmischen Verwendung gemäß Anspruch 7 oder 8, welche die folgende Zusammensetzung in Prozent auf das Gesamtgewicht aufweist:
| | |
|---|---|
| Ubichinon | 0,05 - 0,5 |
| Vitamin E TPGS | 0,05 - 10 |
| Mg-Ascorbyl-phosphat | 0,05 - 1 |
| wasserlösliche Antioxidantien | ≤ 1. |

10. Eine wässrige Lösung zur ophthalmischen Verwendung gemäß Anspruch 9, wobei die wasserlöslichen Antioxidantien Cystein und/oder Tyrosin sind.

11. Eine wässrige Lösung zur ophthalmischen Verwendung gemäß einem der Ansprüche 7 bis 10, welche die folgende Zusammensetzung in Prozent auf das Gesamtgewicht aufweist:
| | |
|---|---|
| Ubichinon | 0,05 - 0,5 |
| Vitamin E TPGS | 0,05 - 10 |
| Mg-Ascorbyl-phosphat | 0,05 - 1 |
| wasserlösliche Antioxidantien | ≤ 1 |
| Hydroxymethylcellulose | ≤ 1. |

12. Eine wässrige Lösung zur ophthalmischen Verwendung gemäß Anspruch 11, wobei Hydroxymethylcellulose durch Hydroxypropylcellulose ersetzt ist.

13. Eine wässrige Lösung zur ophthalmischen Verwendung gemäß einem der Ansprüche 7 bis 12, welche die folgende Zusammensetzung in Prozent auf das Gesamtgewicht aufweist:
| | |
|---|---|
| Ubichinon | 0,05 - 0,5 |
| Vitamin E TPGS | 0,05 - 10 |
| Mg-Ascorbyl-phosphat | 0,05 - 1 |
| wasserlösliche Antioxidantien | ≤ 1 |
| Hydroxymethylcellulose | ≤ 1 |
| Mannit | ≤ 1. |

14. Eine wässrige Lösung zur ophthalmischen Verwendung gemäß Anspruch 13, wobei Mannit durch Natriumchlorid (NaCl) ersetzt ist.

15. Eine wässrige Lösung zur ophthalmischen Verwendung gemäß einem der Ansprüche 7 bis 13, welche die folgende Zusammensetzung in Prozent auf das Gesamtgewicht aufweist:
| | |
|---|---|
| Ubichinon | 0,05 - 0,5 |
| Vitamin E TPGS | 0,05 - 10 |
| Mg-Ascorbyl-phosphat | 0,05 - 1 |
| wasserlösliche Antioxidantien | ≤ 1 |
| Hydroxymethylcellulose | ≤ 1 |
| Mannit | ≤ 1 |
| Benzalkoniumchlorid | ≤ 0,01. |

16. Eine wässrige Lösung zur ophthalmischen Verwendung gemäß einem der Ansprüche 7 bis 14, welche die folgende Zusammensetzung in Prozent auf das Gesamtgewicht aufweist:
| | |
|---|---|
| Ubichinon | 0,05 - 0,5 |
| Vitamin E TPGS | 0,05 - 10 |
| Mg-Ascorbyl-phosphat | 0,05 - 1 |
| wasserlösliche Antioxidantien | ≤ 1 |
| Natriumchlorid | ≤ 1 |
| Benzalkoniumchlorid | ≤ 0,01 |
| Borsäure und/oder Natriumborat ≤ | 0,1 |
| Dinatrium-edetat | ≤ 0,1. |

17. Emulsion zur ophthalmischen Verwendung gemäß einem der Ansprüche 1 bis 4, welche die folgende Zusammensetzung in Prozent auf das Gesamtgewicht aufweist:
| | |
|---|---|
| Ubichinon | 0,05 - 0,5 |
| Vitamin E TPGS | 0,05 - 10 |
| Mg-Ascorbyl-phosphat | 0,05 - 1 |
| wasserlösliche Antioxidantien | ≤ 1 |
| Hydroxymethylcellulose | ≤ 1 |
| Mannit | ≤ 1 |
| pflanzliche Öle | ≤ 5. |

18. Emulsion zur ophthalmischen Verwendung gemäß Anspruch 16, wobei die pflanzlichen Öle Mandelöl, Reisöl, Sojaöl sind.

19. Verwendung von Ubichinon oder Ubichinol in Verbindung mit Vitamin E TPGS gemäß einem der Ansprüche 2 bis 4 zur Herstellung einer wässrigen Lösung zur ophthalmischen Verwendung wie in einem der Ansprüche 7 bis 16 beschrieben.

20. Verwendung von Ubichinon oder Ubichinol in Verbindung mit Vitamin E TPGS gemäß einem der Ansprüche 2 bis 4 zur Herstellung einer wässrigen Emulsion zur ophthalmischen Verwendung wie in einem der Ansprüche 17 oder 18 beschrieben.

## Revendications

1. Utilisation de vitamine E sous forme TPGS en tant qu'agent solubilisant pour ubiquinone ou ubiquinol, en vue de la préparation de solutions aqueuses ou d'émulsions aqueuses de ladite ubiquinone ou dudit ubiquinol.

2. Solution ou émulsion aqueuse d'ubiquinone ou d'ubiquinol, en association avec de la vitamine E sous forme TPGS, pour utilisation en tant que médicament ophtalmique servant à réduire les effets du stress oxydant au niveau des yeux.

3. Solution ou émulsion aqueuse d'ubiquinone ou d'ubiquinol, en association avec de la vitamine E sous forme TPGS, pour utilisation en tant que médicament ophtalmique servant à traiter des manifestations pathologiques en relation avec l'apoptose sur la fraction des kératinocytes de la cornée.

4. Solution ou émulsion aqueuse d'ubiquinone ou d'ubiquinol, en association avec de la vitamine E sous forme TPGS, pour utilisation en tant que médicament ophtalmique servant à traiter des pathologies dégénératives, y compris les ophtalmopathie ischémique, opacité sénile des cristallins, cataracte, uvéite, ainsi que les pathologies dégénératives familiales héréditaires, de dérèglement métabolique ou séniles liées à l'âge.

5. Solution ou émulsion aqueuse d'ubiquinone ou d'ubiquinol, en association avec de la vitamine E sous forme TPGS, pour utilisation en tant que médicament ophtalmique servant à traiter des pathologies neurodégénératives de la rétine et du nerf optique, y compris les glaucome, dégénérescence maculaire liée à l'âge, rétinite pigmentaire, maladie de Stargardt, kystes maculaires vitelliformes, dystrophie des cônes, rétinopathie diabétique proliférante, rétinopathie hypertensive, décollement de la rétine, rétinoblastome, ainsi que les névrites et neuropathies optiques d'origine toxique, inflammatoire ou dégénérative.

6. Solution ou émulsion aqueuse d'ubiquinone ou d'ubiquinol, en association avec de la vitamine E sous forme TPGS, pour utilisation en tant que médicament ophtalmique, conforme à l'une des revendications 2 à 5, dans laquelle ladite ubiquinone est le co-enzyme Q10 ou l'ubiquinol correspondant.

7. Solution aqueuse pour utilisation ophtalmique, conforme à l'une des revendications 2 à 6, contenant une ubiquinone en une quantité représentant de 0,05 à 0,5 % de son poids total et de la vitamine E sous forme TPGS en une quantité représentant de 0,05 à 10 % de son poids total, ainsi que des excipients pharmacologiquement compatibles.

8. Solution aqueuse pour utilisation ophtalmique, conforme à la revendication 7, présentant la composition suivante, en pourcentages rapportés à son poids total :
| | |
|---|---|
| ubiquinone | 0,05 - 0,5 |
| vitamine E (TPGS) | 0,05 - 10 |
| ascorbyl-phosphate de magnésium | 0,05 - 1 |

9. Solution aqueuse pour utilisation ophtalmique, conforme à la revendication 7 ou 8, présentant la composition suivante, en pourcentages rapportés à son poids total :
| | |
|---|---|
| ubiquinone | 0,05 - 0,5 |
| vitamine E (TPGS) | 0,05 - 10 |
| ascorbyl-phosphate de magnésium | 0,05 - 1 |
| anti-oxydants hydrosolubles | ≤ 1 |

10. Solution aqueuse pour utilisation ophtalmique, conforme à la revendication 9, dans laquelle les anti-oxydants hydrosolubles sont de la cystéine et/ou de la tyrosine.

11. Solution aqueuse pour utilisation ophtalmique, conforme à l'une des revendications 7 à 10, présentant la composition suivante, en pourcentages rapportés à son poids total :
| | |
|---|---|
| ubiquinone | 0,05 - 0,5 |
| vitamine E (TPGS) | 0,05 - 10 |
| ascorbyl-phosphate de magnésium | 0,05 - 1 |
| anti-oxydants hydrosolubles | ≤ 1 |
| hydroxyméthyl-cellulose | ≤ 1 |

12. Solution aqueuse pour utilisation ophtalmique, conforme à la revendication 11, dans laquelle l'hydroxyméthyl-cellulose est remplacée par de l'hydroxypropyl-cellulose.

13. Solution aqueuse pour utilisation ophtalmique, conforme à l'une des revendications 7 à 12, présentant la composition suivante, en pourcentages rapportés à son poids total :
| | |
|---|---|
| ubiquinone | 0,05 - 0,5 |
| vitamine E (TPGS) | 0,05 - 10 |
| ascorbyl-phosphate de magnésium | 0,05 - 1 |
| anti-oxydants hydrosolubles | ≤ 1 |
| hydroxyméthyl-cellulose | ≤ 1 |
| mannitol | ≤ 1 |

14. Solution aqueuse pour utilisation ophtalmique, conforme à la revendication 13, dans laquelle le mannitol est remplacé par du chlorure de sodium (NaCl).

15. Solution aqueuse pour utilisation ophtalmique, conforme à l'une des revendications 7 à 13, présentant la composition suivante, en pourcentages rapportés à son poids total :
| | |
|---|---|
| ubiquinone | 0,05 - 0,5 |
| vitamine E (TPGS) | 0,05 - 10 |
| ascorbyl-phosphate de magnésium | 0,05 - 1 |
| anti-oxydants hydrosolubles | ≤ 1 |
| hydroxyméthyl-cellulose | ≤ 1 |
| mannitol | ≤ 1 |
| chlorure de benzalkonium | ≤ 0,01 |

16. Solution aqueuse pour utilisation ophtalmique, conforme à l'une des revendications 7 à 14, présentant la composition suivante, en pourcentages rapportés à son poids total :
| | |
|---|---|
| ubiquinone | 0,05 - 0,5 |
| vitamine E (TPGS) | 0,05 - 10 |
| ascorbyl-phosphate de magnésium | 0,05 - 1 |
| anti-oxydants hydrosolubles | ≤ 1 |
| chlorure de sodium | ≤ 1 |
| chlorure de benzalkonium | ≤ 0,01 |
| acide borique et/ou borate de sodium | ≤ 0,1 |
| sel disodique d'EDTA | ≤ 0,1 |

17. Emulsion pour utilisation ophtalmique, conforme à l'une des revendications 1 à 4, présentant la composition suivante, en pourcentages rapportés à son poids total :
| | |
|---|---|
| ubiquinone | 0,05 - 0,5 |
| vitamine E (TPGS) | 0,05 - 10 |
| ascorbyl-phosphate de magnésium | 0,05 - 1 |
| anti-oxydants hydrosolubles | ≤ 1 |
| hydroxyméthyl-cellulose | ≤ 1 |
| mannitol | ≤ 1 |
| huiles végétales | ≤ 5 |

18. Emulsion pour utilisation ophtalmique, conforme à la revendication 16, dans laquelle les huiles végétales sont de l'huile d'amande, de l'huile de riz et/ou de l'huile de soja.

19. Utilisation d'une ubiquinone ou d'un ubiquinol en association avec de la vitamine E sous forme TPGS, selon l'une des revendications 2 à 4, pour la fabrication d'une solution aqueuse pour utilisation ophtalmique, conforme à l'une des revendications 7 à 16.

20. Utilisation d'une ubiquinone ou d'un ubiquinol en association avec de la vitamine E sous forme TPGS, selon l'une des revendications 2 à 4, pour la fabrication d'une émulsion aqueuse pour utilisation ophtalmique, conforme à la revendications 17 ou 18.
